# FASCICULE DE BREVET EUROPEEN

(11) **EP 3 697 304 B1**
(45) Date de publication et mention de la délivrance du brevet: **23.10.2024**
(21) Numéro de dépôt: 18782120.2
(22) Date de dépôt: 17.09.2018
(51) Int. Cl.: A61B 5/22, A61B 5/11, A61B 5/03, A63B 23/20, A61B 5/00

(54) **DISPOSITIF DE MESURE DES CONTRACTIONS MUSCULAIRES ET/OU DU RELACHEMENT MUSCULAIRE ET MÉTHODES ASSOCIÉES**
VORRICHTUNG ZUR MESSUNG DER MUSKELKONTRAKTIONEN UND / ODER MUSKELENTSPANNUNG UND ZUGEHÖRIGE VERFAHREN
DEVICE FOR MEASURING MUSCLE CONTRACTIONS AND/OR MUSCLE RELAXATION, AND ASSOCIATED METHODS

(30) Priorité: 16.10.2017 FR 1759664
(43) Date de publication de la demande: 26.08.2020
(73) Titulaire: FiziMed, 67000 Strasbourg (FR)
(72) Inventeur: GUAY, Julien, 67000 Strasbourg (FR)
(74) Mandataire: Kessler, Marc
(86) Numéro de dépôt international: PCT/FR2018/052267
(87) Numéro de publication internationale: WO 2019/077215

(56) Documents cités:
- WO-A1-2016/202696
- WO-A1-2017/070787
- US-A- 5 483 832

## Description

### Objet de l'invention

La présente invention se rapporte à un dispositif de mesure des contractions de muscles internes, en particulier des muscles d'une cavité corporelle, plus particulièrement des muscles du périnée, et à une méthode de suivi des contractions et une méthode d'exercice, de ces muscles, qui met en oeuvre le dispositif.

### Etat de la technique

Les dispositifs de stimulation des muscles externes, comme les muscles des bras, des cuisses ou des abdominaux, sont bien connus. Généralement, il s'agit de dispositifs d'électrostimulation délivrant un courant électrique induisant la contraction des muscles.

Pour des muscles internes, en particulier ceux faisant partie d'une cavité musculaire, en particulier pour les muscles du plancher pelvien, il est également connu d'employer des dispositifs dits « actifs », car provoquant des contractions pour exercer et renforcer ces muscles. Toutefois, il existe également des dispositifs dits « passifs » qui ne stimulent pas les muscles, mais enregistrent les contractions naturelles des muscles, et enfin des dispositifs dits « mixtes » combinant à la fois stimulation électrique et enregistrement des contractions.

Par exemple, le document WO2011159906 décrit un dispositif dit « actif », de stimulation du périnée, comprenant une partie extensible, s'étendant à l'aide d'un ballon gonflable, et permettant de mettre en contact des électrodes de surface avec une portion de la paroi vaginale pour provoquer des contractions musculaires.

En outre, le document US2003083590 décrit une sonde intra vaginale « active », pour la stimulation électrique des muscles du périnée, comprenant des moyens de stimulation électrique des muscles et des moyens de communication sans fil pour une communication avec un boitier de contrôle séparé.

Concernant les dispositifs dits « passifs », le document US2016279469 décrit un dispositif d'exercice du périnée, comprenant un corps ovoïde s'insérant dans le vagin, une queue s'étendant à l'extérieur et comprenant une antenne de communication pour l'interaction avec un dispositif externe du type Smartphone, les deux parties étant réunies par une partie resserrée comprenant des excroissances d'ancrage et une jauge de contrainte pour la détection des contractions, la jauge pouvant s'étendre dans le corps ovoïde et dans la queue.

Le document WO2016/202696 décrit une sonde de mesure de la force des muscles du plancher pelvien, comprenant une coque pleine, recouverte d'une couche d'un matériau biocompatible, et comprenant une pluralité d'éléments capteurs et une unité de traitement, qui sont noyées dans un matériau polymérique conducteur et déformable, la sonde ayant une forme sensiblement cylindrique, et dont certaines régions ont des diamètres de section transversales de différentes tailles.

Le document US5483832 décrit une sonde de mesure de contractions, sensiblement cylindrique, comprenant une pluralité de chambres cylindriques déformables, disposées de façon concentrique les unes sur les autres, et connectées à un conduit rempli d'un fluide, le conduit étant connecté à un dispositif d'affichage.

Toutefois ce type de dispositif présente l'inconvénient de ne pas être sensible et de ne permettre la détection de contraction des muscles qu'au niveau d'une zone très restreinte de la cavité corporelle dans laquelle se trouvent ces muscles.

Le document WO2016042310 décrit un dispositif de mesure de la contraction des muscles du périnée, de forme ovoïde, comprenant un boitier comprenant une première partie supérieure et une seconde partie comprenant une partie inférieure et une partie centrale, un doigt faisant saillie à l'intérieur du boitier depuis la partie supérieure dans un puits de guidage de la partie centrale, pour, en réponse à une force exercée sur la partie supérieure et/ou inférieure, exercer une pression sur un capteur de force disposé dans la partie inférieure du boitier.

Néanmoins, ce dispositif présente l'inconvénient d'être peu sensible, ce qui nécessite l'emploi de moyens d'amortissement internes au dispositif, disposés entre la fin du doigt et le capteur de force, afin d'améliorer la stabilité des mesures. Par ailleurs, ce dispositif est sujet au déplacement, en rotation, des parties supérieure et inférieure l'une par rapport à l'autre, ce qui entraine un mauvais positionnement du doigt dans le puits et donc un manque de fiabilité des mesures. Cela nécessite, par conséquent, l'emploi d'un amortisseur de rotation, une barre anti roulis, pour réduire ou empêcher ces déplacements intempestifs. Au final, un tel dispositif est complexe à fabriquer, peu fiable et peu sensible lors des mesures de contractions.

Parmi les dispositifs dits « mixtes», le document WO2016203485 décrit un dispositif cylindrique, ayant une longueur de 3 à 8 cm et un diamètre compris entre 1 et 3 cm, comprenant un capteur de pression du type piézoélectrique, disposé à une ou les deux extrémités du dispositif pour détecter des forces radiales exercée autour de la circonférence du dispositif, une batterie d'alimentation électrique, un émetteur pour communiquer avec un récepteur et un système de stimulation musculaire électronique.

De même, le document WO2017/070787 décrit un dispositif mixte dont le corps comprend un squelette interne comprenant une surface externe, recouverte d'une couche externe de matériau non toxique, flexible, et formant avec la surface externe du squelette un canal d'écoulement d'air partiellement compressible et en communication de fluide avec au moins un capteur de pression disposé dans le corps du dispositif, pour détecter des changements de pression d'air dus aux pressions appliquées sur la couche externe de matériau flexible.

Le document ES201630892U divulgue un dispositif de mesure des contractions et/ou du relâchement d'un ou plusieurs muscles d'une cavité corporelle comprenant un capteur de pression positionné à l'extérieur du corps principal.

### Buts de l'invention

La présente invention vise à fournir un dispositif de mesure des contractions naturelles, et le relâchement, de muscles d'une cavité corporelle et une méthode de mesure, une méthode de suivi, des contractions et leur relâchement, et une méthode d'exercice de tels muscles, qui ne présentent pas les inconvénients de l'état de la technique.

La présente invention vise, en particulier, à fournir un dispositif de mesure des contractions naturelles, et le relâchement, de muscles d'une cavité corporelle qui présente une sensibilité de mesure améliorée, et permettant de détecter la contraction, et également le relâchement, d'un muscle quel que soit l'endroit de la cavité corporelle où elle survient.

La présente invention vise, également, à fournir un dispositif de mesure des contractions naturelles de muscles d'une cavité corporelle qui est moins complexe à fabriquer.

La présente invention propose également une méthode du suivi de la contraction, et le relâchement, des muscles d'une cavité corporelle et une méthode d'exercice de ces muscles, qui ne présentent pas les inconvénients des solutions de l'état de la technique, et qui soient simples à mettre en oeuvre.

### Résumé de l'invention

La présente invention porte sur un dispositif de mesure des contractions et/ou du relâchement d'un ou plusieurs muscles d'une cavité corporelle, le dispositif comprenant un corps creux venant se positionner dans la cavité corporelle, et recouvert d'un revêtement fait, ou comprenant, un matériau bio compatible, le corps étant constitué de deux demi coques, chacune étant liée physiquement, de façon permanente et continue lors de l'utilisation du dispositif, à l'aide de moyens de liaison non compressibles ou déformables, à au moins un capteur de pression, ou une partie du capteur de pression, disposé dans le corps du dispositif.

Selon des modes préférés de l'invention, le dispositif selon l'invention comprend au moins une, ou une combinaison quelconque appropriée, des caractéristiques suivantes :
- les moyens de liaison des demi coques au capteur comprennent, ou sont constituées par, au moins une vis, de préférence deux vis, engageant à la fois une des demi coques et le capteur ou une partie dudit capteur,
- les demi coques sont montées flottantes l'une par rapport à l'autre, les bords de la première demi coque n'entrant pas en contact avec les bords de la seconde demi coque, créant ainsi un espace entre les deux demi coques,
- ledit dispositif comprend deux capteurs de pression, chaque capteur étant relié, par des moyens de liaison distincts, à une demi coque,
- le ou les capteurs de pression est, ou sont, du type peson et comprend, ou comprennent, un corps fait d'aluminium et deux jauges de contraintes disposées sur deux surfaces opposées du corps,
- le dispositif comprend en outre des moyens de communication sans fil avec un dispositif mobile externe,
- lequel corps comprend une première partie se trouvant, lors de l'utilisation dudit dispositif, dans la cavité corporelle forme, qui est de forme ovoïde, une seconde partie se trouvant, lors de l'utilisation dudit dispositif, hors de la cavité corporelle, de forme sphéroïde, ladite première et ladite seconde partie étant reliées entre elles par une partie intermédiaire de forme sensiblement cylindrique.

La présente invention porte également sur un ensemble de mesure de suivi des contractions et/ou du relâchement et d'exercice des muscles formant, ou étant compris dans une cavité corporelle, l'ensemble comprenant un ou plusieurs dispositifs de mesure selon l'invention et un ou plusieurs dispositifs mobiles externes avec lequel ou lesquels le ou les dispositifs selon l'invention communique(nt).

Dans l'ensemble de mesure selon l'invention, le ou les dispositifs mobiles externes comprend ou comprennent des moyens de communication avec l'utilisateur ou l'utilisatrice du ou des dispositifs de mesure selon l'invention, et éventuellement également des moyens de communication utilisant un réseau de communication téléphonique ou Internet pour une communication avec un tiers distant.

La présente invention porte aussi sur une méthode de mesure de la contraction et/ou du relâchement des muscles d'une cavité corporelle d'un utilisateur, comprenant les étapes de prendre un ou plusieurs dispositifs de mesure selon l'invention, ou l'ensemble de mesure selon l'invention, de mettre en marche le ou les dispositifs de mesure, de placer le ou les dispositifs de mesure dans une cavité corporelle de l'utilisateur comprenant ou étant formée de muscles, d'identifier et de mesurer l'amplitude et/ou de la durée d'une première contraction maximale et/ou d'un premier relâchement maximal des muscles, d'identifier et de mesurer au cours du temps, l'amplitude et/ou la durée et/ou la fréquence d'une ou plusieurs contractions ultérieures et/ou relâchements ultérieurs, l'amplitude et/ou de la durée de la première contraction maximale et l'amplitude et/ou la durée et/ou la fréquence de la ou des contractions ultérieures et/ou relâchements ultérieurs constituant des données de mesures, et de communiquer et de présenter, en temps réel, à l'utilisateur les données de mesures.

De préférence la méthode de mesure selon l'invention comprend en outre une étape de communication par le ou les dispositifs de mesure de son ou leur état de fonctionnement et/ou une étape de calibration du ou desdits dispositifs de mesure, avant et/ou après sa ou leur mise en place dans la cavité corporelle.

La présente invention porte, en outre, sur une méthode de suivi de la contraction et/ou du relâchement, des muscles d'une cavité corporelle comprenant la mise en oeuvre de la méthode de mesure selon l'invention, et une étape de comparaison des données de mesures, collectées au cours du temps à l'aide de la méthode de mesure selon l'invention, avec les données de la première contraction maximale et/ou du relâchement maximal, pour une même série de mesures, ou la comparaison avec les données des premières contractions maximales et/ou de relâchements maximaux de séries de mesures précédentes d'un historique de mesures ou d'une ou plusieurs valeurs moyennes de premières contractions maximales et/ou relâchements maximaux desdites mesures précédentes.

De préférence, la méthode de suivi de la contraction des muscles comprend en outre une étape de communication à l'utilisateur d'une ou plusieurs représentations écrites, graphiques et/ou auditives, des résultats issus de l'étape de comparaison des données.

La présente invention porte, de plus, sur une méthode d'exercice de muscles d'une cavité corporelle comprenant la mise en oeuvre de la méthode de mesure selon l'invention, ou de la méthode de suivi selon l'invention, et comprend une étape d'adaptation de l'amplitude et/ou la durée et/ou le nombre et/ou fréquence des contractions et/ou relâchements effectuées par, ou demandées à, l'utilisateur en fonction des données de la première contraction maximale et/ou premier relâchement maximal et/ou de la fatigue musculaire de l'utilisateur identifiée par la méthode de suivi de l'invention.

De préférence, la méthode de mesure selon l'invention et/ou la méthode de suivi selon l'invention et/ou la méthode d'exercice selon l'invention, comprend ou comprennent en outre une étape de communication du ou des dispositifs de mesure selon l'invention, ou du dispositif mobile externe, avec un tiers distant de l'utilisateur pour la préparation et/ou l'exécution d'une ou plusieurs étapes des méthodes de mesure, de suivi et/ou d'exercice, ou pour la communication d'indicateurs représentatifs de la performance musculaire de l'utilisateur.

### Brève description des figures

La figure 1 est une représentation schématique d'une vue de coté d'un mode de réalisation du dispositif selon l'invention.
La figure 2 est une représentation schématique d'une vue en perspective du mode de réalisation du dispositif de la figure 1.
La figure 3 est une représentation schématique d'une vue en coupe longitudinale du mode de réalisation du dispositif de la figure 1.
La figure 4 est une représentation schématique d'une vue éclatée, et en perspective, du corps du mode de réalisation du dispositif de la figure 1.

### Description détaillée de l'invention

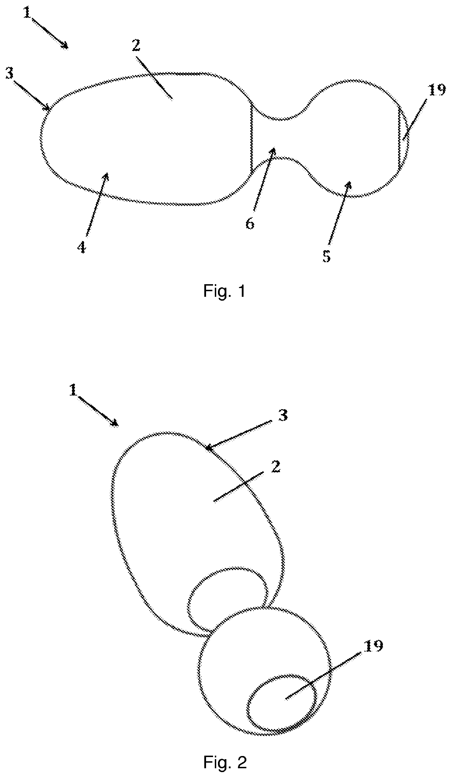

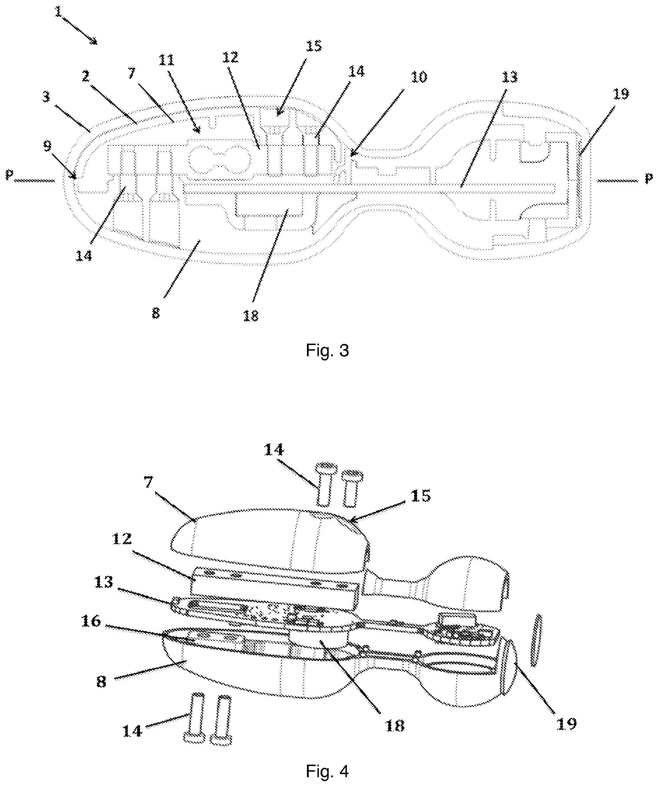

Dans la suite de la description et des revendications, les termes « haut », « bas », « supérieur », « inférieur », « vertical » ou « horizontal » font référence à la position horizontale du dispositif 1 selon l'invention, comme représenté aux figures 1 et 3.

Le dispositif 1 selon l'invention est une sonde dite « passive » car elle ne stimule pas les muscles, mais enregistre leurs contractions naturelles et/ou le relâchement musculaire faisant suite à une contraction, provenant de la stimulation volontaire des muscles par l'utilisateur ou utilisatrice du dispositif 1.

Le dispositif 1 comprend un corps 2 creux, de préférence fait en matériau rigide, de préférence plastique, et avantageusement recouvert d'un matériau ayant la forme d'un revêtement 3 ou d'un film, fait d'un seul tenant, ou bien de plusieurs éléments, et fait d'un matériau de préférence à base d'une matière bio compatible, par exemple à base de silicone de grade médical. Pour un revêtement 3 en plusieurs éléments, la jonction de ces éléments entre eux peut se faire bords à bords, à l'aide par exemple d'une colle bio compatible, par un ajustement mécanique ou bien l'emploi de moyens mécaniques de jonction, par exemple des clips. De préférence, la jonction des éléments permet de garantir l'étanchéité du dispositif 1. Le revêtement 3 est de préférence lisse et sans aspérité afin de faciliter le nettoyage et l'entretien du dispositif 1.

Le corps 2 du dispositif 1 a des dimensions et une forme adaptées et compatibles avec la ou les cavités corporelles dans laquelle, ou lesquelles, il est introduit pour mesure la contraction du ou des muscles formant, ou étant compris, dans la ou les cavités corporelles.

Dans la suite de la description, le dispositif 1 selon l'invention sera décrit pour une cavité corporelle ouverte sur l'extérieur, et tel que représentée aux figures 1 à 4. Toutefois, le dispositif 1 peut être adapté à tous types et toute nature de cavités corporelles possibles.

Ainsi, pour une telle cavité corporelle, par exemple le vagin d'une femme ou la cavité anale d'une femme ou d'un homme, le corps 2 du dispositif 1 comprend une première partie 4 se trouvant, lors de l'utilisation du dispositif 1, dans ladite cavité corporelle et une seconde partie 5 se trouvant hors de la cavité corporelle, ces deux parties 4 et 5 étant reliées entre elles par une partie intermédiaire 6 (figure 1).

La première partie 3 du dispositif 1 a une forme et des dimensions qui sont fonction de, et adaptées à, la ou les cavités corporelles dans laquelle ou lesquelles elle est destinée à être introduite. De préférence, cette première partie 3 a une forme sensiblement cylindrique, sphéroïde, ou sensiblement la forme d'une sphère, ovoïde ou bien ellipsoïde.

La seconde partie 5 du dispositif 1 a une forme et des dimensions adéquates qui permettent sa préhension par l'utilisateur pour la mise en place dans, et l'extraction du dispositif 1 hors, de la cavité corporelle ouverte vers l'extérieur. Cette seconde partie 5 a de préférence une forme sphéroïde, de préférence elle a sensiblement la forme d'une sphère, ou avantageusement une forme ovoïde, et peut comprendre tout ou partie des composants du dispositif 1.

La partie intermédiaire 6 a toute forme et dimensions adéquates. De préférence, elle est cylindrique, ou sensiblement cylindrique, et forme un rétrécissement entre la première partie 3 et la seconde partie 4 du corps 2, ce qui présente l'avantage de procurer un confort amélioré lors de l'utilisation du dispositif 1, en particulier pour ce qui concerne la ou les cavités corporelles fermées par un sphincter, des tissus ou muscles, comme par exemple l'orifice anal ou vaginal.

Le corps 2, ou pour un dispositif 1 comprenant plusieurs parties, au moins la partie du corps devant se trouver dans la cavité corporelle, à savoir la première partie 4 du corps 2, comprend, ou est fait ou faite, de deux demi coques 7 et 8 creuses, formant une partie supérieure et une partie inférieure dudit corps 2 ou de ladite première partie 4 du corps 2, et qui de préférence sont creuses ou partiellement creuses. De préférence, les deux demi coques 7 et 8 comprennent également, et forment respectivement la partie supérieure et la partie inférieure de la seconde partie 5 et de la partie intermédiaire 6 du corps 2 (figure 4).

Les deux demi coques 7 et 8 ont une forme et des dimensions adaptées et compatibles avec la forme et les dimensions de la ou des cavités corporelles dans laquelle ou lesquelles le corps 2 doit se positionner. De préférence, les deux demi coques 7 et 8 ont la même forme et les mêmes dimensions ; toutefois, il est envisageable que les deux demi coques 7 et 8 aient une forme différente.

Par exemple, et si la cavité corporelle est le vagin et/ou la cavité anale, les deux demi coques 7 et 8 ont la même forme, afin que le corps 2, ou au moins la première partie 4 du corps 2, ait une forme sensiblement cylindrique, ou sphéroïde, ou ayant sensiblement la forme d'une sphère, ovoïde ou bien une forme ellipsoïde. Un corps 2, ou première partie 4 du corps 2, de forme ovoïde (figures 1 à 4), présente l'avantage d'une insertion plus facile et d'être plus confortable lors de l'utilisation du dispositif 1. Néanmoins, il est possible qu'au moins une des demi coques 7 ou 8 présente une surface convexe et qu'elle ait une forme cylindrique ou sensiblement cylindrique, sphéroïde, ovoïde ou ellipsoïde, et l'autre demi coque 7 ou 8 présente une surface sensiblement plane. Il est également envisageable qu'une demi coque 7 ou 8 ait une forme cylindrique, sphéroïde, ellipsoïde et que l'autre ait une forme ovoïde.

A titre d'exemple et pour une cavité corporelle qui est le vagin, la première partie 4 du corps est de forme ovoïde avec un diamètre de la partie la plus large compris entre 2,5 et 4,0 cm et d'une longueur comprise entre 5 et 7 cm, la seconde partie de forme sphéroïde ayant un diamètre compris entre 2 et 4 cm, pour une longueur totale du dispositif 1 compris entre 8 et 11 cm.

Dans un mode de réalisation préféré de l'invention, les deux demi coques 7 et 8 entrent en contact l'une avec l'autre bords à bords. De préférence, elle sont rigides, tout en étant déformables sous la pression exercée par le ou les muscles dont les contractions sont à mesurer, et sont également résilientes.

Dans un autre mode de réalisation préféré de l'invention, au moins une de deux demi coques 7 ou 8, avantageusement les deux demi coques 7 et 8, est ou sont rigide(s) et sensiblement non déformable(s) sous la force de contraction du ou des muscles. La ou les deux demi coques 7 ou 8 est ou sont montée(s) flottante(s) l'une par rapport à l'autre. En d'autres termes, les bords de la première demi coque 7 n'entrent pas en contact avec les bords de la seconde demi coque 8, créant ainsi un espace 9 entre les deux demi coques 7 et 8. Ceci présente l'avantage d'obtenir un espace d'amortissement, dont le débattement est de préférence limité par des butées placées sur au moins une des demi coques 7 ou 8, permettant ainsi d'empêcher un déplacement vertical trop important des demi coques 7 et 8 l'un par rapport à l'autre qui pourrait endommager le ou les capteurs 11.

De même, et pour la première partie 4 du corps 2, il est avantageux de prévoir que les bords de chaque demi coques 7 et 8 de cette première partie 4 n'entrent pas en contact avec le ou les bords de la partie intermédiaire 6 du corps 2, ou de la demi coque 7 ou 8 de cette partie intermédiaire 6, créant ainsi un espace 10 entre la première partie 4 et la partie intermédiaire 6 du corps 2. Ceci présente l'avantage de créer un espace d'amortissement, qui peut, de préférence être limité par des butées placées sur au moins une des demi coques 7 ou 8.

Le dispositif 1 comprend en outre au moins un capteur 11 de pression, de préférence deux capteurs 11, un par demi coque 7 ou 8, disposé(s) à l'intérieur du corps 2.

Le ou les capteurs 11 sont de préférence du type peson.

Dans des modes de réalisation particuliers de l'invention, le ou les capteurs 11 comprend ou comprennent un corps 12, fait d'un matériau rigide, mais déformable, de préférence déformable sous l'action d'une pression exercée par la contraction et/ou le relâchement, du ou des muscles de la ou des cavités corporelles. Le matériau rigide peut, par exemple, être fait ou comprendre de l'aluminium.

Le corps 12 comprend deux jauges de contraintes mesurant les moindres déformations, même infimes, du corps 12 du capteur 11. De préférence, les jauges de contraintes sont disposées sur au moins la face, avantageusement les deux faces, du corps 12 qui subit ou subissent les déformations issues de la pression de contraction, permettant ainsi, lors de la déformation du corps 12 sous l'action d'une pression exercée sur l'une et/ou l'autre face, de mesurer l'élongation des deux jauges, qui est fonction de la pression exercée, et/ou de mesurer le retour à l'état de repos des deux jauges après avoir subi l'élongation, qui est fonction du relâchement après la pression. De préférence, les jauges de contraintes sont disposées sur la surface des deux faces opposées du corps 12, de part et d'autre du corps 12.

Ce type de capteurs 11 présente l'avantage de pouvoir détecter, et donc mesurer, de façon sensible une force de compression appliquée sur n'importe quel endroit de la surface externe du corps 2 du dispositif 1 et au moins de la première partie 3 du corps 2 et/ou la force de relâchement faisant suite à la compression.

De préférence, le ou les capteurs 11 enregistre les forces de compression et/ou de relâchement à une fréquence comprise entre cinq et quinze fois par seconde (5 à 15Hz), avantageusement à une fréquence de cent milli secondes (10Hz).

Le ou les capteurs 11 sont disposés sur, et sont en relation électrique avec, des moyens de support 13, par exemple une carte électronique, une carte sur laquelle sont également fixés, ou reliés électriquement, les autres composants électroniques ou électriques faisant partie du dispositif 1. Ces moyens 13 de support, cette carte électronique, sont de préférence disposés sensiblement de façon centrale par rapport au corps 2, au niveau d'un plan P, qui peut être le plan de jointure des demi coques 7 et 8 ou le plan de symétrie du corps 2, ou bien dans un plan sensiblement parallèle au plan P (figure 3). De préférence, ces moyens 13 de support, cette carte électronique, peuvent s'étendre le long de la première partie 3 ou également le long de la partie intermédiaire 6 et seconde partie 5 du corps 2 (figure 3).

Le ou les capteurs 11 est, ou sont, en relation avec la demi coque 7 ou 8 correspondante, seulement par l'intermédiaire de moyens 14 de liaison.

Ces moyens 14 de liaison ne sont pas, ne comprennent pas et ne mettent pas en oeuvre, un ou plusieurs fluides, liquides ou gaz, ni de l'air.

Ces moyens 14 de liaison sont rigides, sensiblement non compressibles ou déformables, en tout état de cause pas compressibles ou déformables sous la pression de contraction et/ou de relâchement, du ou des muscles de la ou des cavités corporelles que le dispositif 1 est censé mesurer.

Ces moyens 14 lient physiquement et mécaniquement, de façon permanente et continue, la ou chacune des demi coques 7 ou 8, au capteur 11, de préférence chaque demi coque 7 et 8 à un capteur 11, ou chaque demi coques 7 ou 8 à son capteur 11, et permettent la transmission de la force exercée sur la surface d'une ou des deux demi coques 7 et 8 au capteur 11 ou à chacun des capteurs 11.

Ces moyens 14 comprennent, ou sont constitués par, au moins une vis, de préférence deux vis, avantageusement faites ou comprenant du plastique rigide ou du métal, engageant un orifice 15, ou trou débouchant, pratiqué dans la ou les demi coques 7 et/ou 8, et éventuellement engageant également une partie taraudée 16 de la demi coque 7 ou 8, les moyens 14 engageant également un orifice 17 taraudé, qui est éventuellement également un trou débouchant, pratiqué dans le corps 12 du ou des capteurs 11.

Les moyens 14 et leur liaison permanente d'assujettissement avec la ou les demi coques 7 et 8 et le ou les capteurs 11 présentent l'avantage de ne pas avoir de pièces mobiles, en mouvement, lors de l'utilisation du dispositif 1 selon l'invention.

Que le corps 2 soit rigide et déformable, ou que les demi coques 7 et 8 soient montées flottantes l'une par rapport à l'autre, la force de compression exercée sur le corps 2 sera transmise directement au(x) capteur(s) 11, sans perturbations qui pourraient être induites par des mouvements parasites ou des déplacements d'une ou des deux demi coques 7 et 8 l'une par rapport à l'autre ou de la première partie 3 du corps 2 par rapport à la seconde partie 5 et la partie intermédiaire 6. Le dispositif 1, comme les mesures faites, s'en trouvent plus fiables.

Le mode de réalisation dans lesquels les demi coques sont flottantes présente l'avantage supplémentaire d'avoir un dispositif 1 d'une sensibilité améliorée sur l'ensemble du corps 2, en particulier sur la première partie 3 du corps 2, la moindre contraction, et également le moindre relâchement, du ou des muscles de la ou des cavités corporelles étant transmises directement au(x) capteur(s) 11. De plus, cela présente l'avantage d'avoir un dispositif 1 adaptable aux forces de compression maximales pouvant s'exercer sur le corps 2, et donc adaptable aux cavités corporelles et également aux différent utilisateur, cette adaptation se faisant en variant la dimension de l'espace 9.

Le dispositif 1 comprend en outre des moyens 18 d'alimentation en électricité le ou les capteurs 11, mais également tous les éléments qui composent le dispositif 1. Ces moyens 18 d'alimentation en électricité comprennent, ou sont, une pile ou de préférence une batterie rechargeable.

Dans les modes de réalisation du dispositif 1 dans lesquels les moyens 18 d'alimentation en électricité comprenant une batterie rechargeable, le dispositif 1 peut comprendre en outre des moyens 19 pour recharger la batterie. Ces moyens 19 peuvent être filaires ou sans fil ; ils peuvent par exemple mettre en oeuvre un phénomène d'induction. De préférence, ces moyens 19 sont disposés à l'extrémité du corps 2, en particulier à l'extrémité de la seconde partie 5 du corps 2 qui est opposée à la première partie 3.

Les moyens 18 d'alimentation en électricité peuvent également comprendre, ou coopérer avec différents capteurs ou sondes permettant de suivre ou de s'assurer de la charge ou du chargement de la batterie, y compris une sonde de température afin d'éviter les surchauffes durant la charge de la batterie.

Les moyens 18 d'alimentation en électricité peuvent également comprendre, ou coopérer avec des moyens de régulation de la tension en électricité délivrée aux composants du dispositif 1.

Le dispositif 1 comprend en outre des moyens de mise en marche et d'arrêt. Classiquement, il peut s'agir d'un bouton poussoir, avantageusement disposé au niveau de la seconde partie 5 du corps 2. Toutefois, de préférence, ces moyens comprennent un accéléromètre détectant une certaine amplitude d'un mouvement du dispositif 1 permettant la mise en marche de ce dernier. Avantageusement, ces moyens comprennent des moyens de temporisation permettant l'arrêt, ou la mise en veille, du dispositif 1 après un lapse de temps suivant le dernier mouvement du dispositif 1 détecté.

Le dispositif 1 peut comprendre en outre des moyens de communication visuelle de son état, en marche, éteint, en veille, et/ou du niveau de charge de la pile ou de la batterie. Ils peuvent comprendre, ou être constitués, d'une ou plusieurs LED, éventuellement de couleur, et pouvant être clignotantes.

De préférence, le dispositif 1 comprend des moyens d'appareillement à un ou plusieurs dispositifs externes, distincts du dispositif 1 selon l'invention. Il comprend également des moyens de communication sans fil, par exemple Bluetooth^{®} de type « faible énergie », pour une communication avec un ou plusieurs autres dispositifs 1 selon l'invention et/ou avec un ou plusieurs dispositifs externes. Ces moyens de communication sont, de préférence, disposés dans la seconde partie 5 du corps 2, celle se trouvant à l'extérieur de la cavité corporelle durant l'utilisant du dispositif 1.

Le dispositif 1 peut comprend en outre des capteurs, par exemple une sonde de température, afin de mesurer, et suivre dans le temps, la température de la cavité corporelle dans laquelle le dispositif 1 a été introduit.

Comme décrit précédemment, le dispositif 1 peut comprendre un accéléromètre pouvant faire partie des moyens de mise en marche et d'arrêt du dispositif 1, néanmoins, cet accéléromètre permet également de lier une force de pression et/ou de relâchement détectée par le ou les capteurs 11 de pression avec un mouvement de la cavité corporelle et donc une contraction et/ou un relâchement des muscles. Cela présente l'avantage de gagner en précision dans la détection des contractions et/ou des relâchements.

Le dispositif 1 peut comprendre en outre un ou plusieurs capteurs d'orientation, un gyroscope et une boussole numérique, éventuellement couplés avec l'accéléromètre afin d'obtenir la position du dispositif 1 dans les trois dimensions de l'espace formé par la cavité corporelle et donc, non seulement de détecter un mouvement de contraction et/ou de relâchement des muscles, mais également d'identifier sa direction dans l'espace.

Le dispositif 1 peut comprendre en outre des moyens d'auto calibration.

Le dispositif 1 peut comprendre en outre des moyens matériels ou logiciels afin de mettre en oeuvre la méthode de mesure, la méthode de suivi des contractions et/ou du relâchement musculaire et la méthode d'exercice selon la présente invention. A ces fins, il peut comprendre un ou plusieurs microprocesseurs, une mémoire et des moyens de stockage des instructions de programmation ou de fonctionnement du dispositif 1.

Le dispositif 1 peut avantageusement faire partie d'un ensemble pour la mesure de contractions et/ou du relâchement et/ou l'exercice des muscles d'une cavité corporelle, qui comprend un ou plusieurs autres dispositifs 1 selon l'invention, et qui comprend en outre au moins un dispositif externe avec lequel le dispositif 1 selon l'invention communique.

Ce dispositif externe comprend des moyens de communication avec le ou les dispositifs 1 selon l'invention, des moyens de communication avec l'utilisateur ou l'utilisatrice du ou des dispositifs 1, et éventuellement également des moyens de communication, utilisant par exemple un réseau téléphonique ou Internet, pour une communication avec une entité distante de l'utilisateur ou utilisatrice du dispositif 1 selon l'invention, par exemple un serveur informatique. Le dispositif externe comprend en outre des moyens de réception, des moyens de traitement et des moyens d'interprétation, des données et mesures provenant, ou en direction, du ou des dispositifs 1. Ces moyens peuvent être, ou comprendre, des moyens matériels et/ou logiciels. En particulier, ce dispositif externe peut comprendre un microprocesseur, un récepteur apte à recevoir des données, une mémoire, et une interface utilisateur graphique, telle qu'un écran, et des touches, ou un écran tactile.

Le dispositif externe est de préférence mobile. Il peut s'agir d'un micro-ordinateur ou ordinateur portable, mais de préférence, il s'agit d'un dispositif mobile de communication, apte à communiquer via un réseau de communication, téléphonique ou informatique, en particulier apte à communiquer par Internet, comme par exemple un téléphone, un Smartphone, une tablette électronique ou tous dispositifs équivalents.

Le dispositif 1 selon l'invention, ou l'ensemble pour la mesure des contractions et/ou le relâchement des muscles dont il fait partie, est de préférence utilisé pour mesurer les contractions et/ou le relâchement des muscles formant, ou étant compris, dans une cavité corporelle, cette cavité corporelle étant avantageusement un vagin ou la cavité anale. Le dispositif 1 permet de mesurer la vitesse et/ou l'amplitude à laquelle ou auxquelles les contractions ou les relâchement s'effectuent. Le dispositif 1 peut également être utilisé pour suivre, au cours du temps, les contractions et/ou le relâchement de tels muscles, les exercer ou les renforcer.

De préférence, le dispositif 1 selon l'invention, ou l'ensemble pour la mesure des contractions et/ou le relâchement des muscles dont il fait partie, est utilisé pour fournir, à l'utilisateur dudit dispositif 1 ou dudit ensemble, des informations sur les contractions et/ou le relâchement des muscles sous observation, une information, de préférence délivrée par le dispositif externe, qui peut être visuelle et prendre par exemple la forme d'un ou plusieurs messages écrits ou graphique, ou bien encore auditive.

La méthode de mesure, selon l'invention, de la contraction naturelle, sans stimulation externe préalable, en particulier provenant du dispositif 1 selon l'invention, et/ou du relâchement des muscles d'une cavité corporelle, comprend la mise en oeuvre d'un ou plusieurs dispositifs 1 selon l'invention ou de l'ensemble de mesure selon l'invention.

La méthode de mesure comprend la mise en marche du ou des dispositifs 1 selon l'invention et son ou leur placement, par l'utilisateur ou l'utilisatrice, dans une cavité corporelle, puis l'identification de la survenue, avant une série de mesures ultérieures, d'une première contraction maximale, la mesure de son l'amplitude et/ou de sa durée, et/ou du relâchement maximal suivant cette première contraction, la mesure de son l'amplitude et/ou de sa durée, ce qui permet de donner une indication de la performance du ou des muscles concernés. La méthode se poursuite ensuite par l'identification de la survenue de contractions ultérieures et/ou de relâchements ultérieurs, et la mesure, au cours du temps, de leur amplitude et/ou de leur durée et/ou de leur fréquence. L'amplitude et/ou de la durée de la première contraction maximale et/ou du premier relâchement maximal, et l'amplitude et/ou de la durée et/ou la fréquence des contractions ultérieures et/ou des relâchements ultérieurs, forment les données de mesures, qui sont communiquées et présentées, de préférence en temps réel, à l'utilisateur ou l'utilisatrice, et qui sont également enregistrées dans le ou les dispositifs 1 de mesure ou le dispositif mobile externe de l'ensemble de mesure.

L'étape de mesure des données de la première contraction maximale et/ou du premier relâchement maximal peut être réalisée automatiquement dès la survenue de la première contraction et/ou du premier relâchement suivant la mise en place du ou des dispositifs 1, à l'initiative de l'utilisateur ou utilisatrice, ou, dans le cas d'une mise en oeuvre de l'ensemble de mesure selon l'invention, en réponse à une demande de contraction musculaire et/ou de relâchement émanant du dispositif mobile extérieur. De même, l'utilisateur ou utilisatrice du ou des dispositifs 1, ou de l'ensemble de mesure selon l'invention, est à l'initiative de la survenue, du nombre et de la fréquence des contractions musculaires et/ou des relâchements. Toutefois, il est également possible d'envisager que le dispositif mobile externe sollicite les contractions et/ou les relâchements, leur nombre et leur fréquence, au travers de messages écrits, graphiques ou auditifs à l'attention de l'utilisateur ou de l'utilisatrice.

Dans le mode de réalisation préféré de la méthode de mesure mettant en oeuvre l'ensemble de mesure selon l'invention, l'étape de communication et de présentation des données se fait par le ou les dispositifs mobiles externes.

La méthode de mesure selon l'invention peut comprendre une étape de communication par le ou les dispositifs 1 de son ou leur état de fonctionnement, à savoir s'il est ou sont en marche ou à l'arrêt, le niveau de charge de leurs moyens d'alimentation électrique, et l'état de l'appareillement et/ou de la communication avec un ou plusieurs dispositifs1 externes.

La méthode de mesure peut comprendre en outre des étapes préalables à la mise en place du ou des dispositifs 1, qui peuvent être mises en oeuvre seules ou en combinaison les unes avec les autres, et qui consistent à :
- communiquer à l'utilisateur ou utilisatrice un manuel d'utilisation du ou des dispositifs 1 et/ou du dispositif externe ;
- vérifier l'intégrité physique du ou des dispositifs 1 selon l'invention, de préférence également, vérifier l'étanchéité du revêtement 3 recouvrant le corps 2 du dispositif ;
- s'assurer du niveau de charge des moyens 18 d'alimentation en électricité du ou des dispositifs 1, et si nécessaire, les recharger ;
- d'étalonnage du ou des dispositifs 1 selon l'invention hors de la ou des cavités corporelles, afin de déterminer le bruit interne induit par le fonctionnement du ou des dispositifs 1, ou de déterminer une valeur moyenne représentative de ce bruit, qui est avantageusement stocké dans le dispositif externe pour une utilisation ultérieure durant les mesures des contractions ou le traitement de ces mesures. De préférence, cet étalonnage se fait lors des recharges des moyens 18 d'alimentation en électricité dans le mode de réalisation de l'invention dans lequel ces moyens 18 comprennent une batterie rechargeable ;
- de corriger, grâce à l'étalonnage ou aux étalonnages du dispositif 1, les erreurs de mesure en lissant les signaux des mesures, éventuellement de détecter et supprimer les mesures erronées.

La méthode de mesure selon l'invention peut en outre comprendre une étape d'étalonnage, ou de calibration, du ou des dispositifs 1 selon l'invention lors de leur présence dans la cavité corporelle. De préférence, cet étalonnage se fait durant une période de temps courte, de l'ordre de quelques millisecondes jusqu'à quelques secondes au maximum, et avantageusement elle est réalisée dès la fin de la mise en place du ou des dispositifs 1 dans la cavité corporelle. Cette étape permet de déterminer : le bruit corporel résiduel de fonctionnement du ou des dispositifs, le bruit issu des contractions, non volontaires, qui pourrait parasiter l'identification et/ou les mesures des contractions volontaires pour une utilisation ultérieure durant les mesures des contractions ou le traitement de ces mesures, et enfin, le seuil de détection d'une contraction et/ou d'un relâchement.

La méthode de mesure selon l'invention peut comprendre une étape de prise en considération des valeurs, ou des valeurs moyennes, des bruits décrits précédemment, pour affiner les mesures en temps réel des contractions volontaires et/ou des relâchements de l'utilisateur ou de l'utilisatrice.

La méthode de suivi de la contraction des muscles d'une cavité corporelle selon l'invention comprend la mise en oeuvre de la méthode de mesure de la contraction et/ou du relâchement des muscles, qui a été décrite précédemment.

La méthode de suivi de la contraction et/ou du relâchement des muscles comprend la comparaison des données de mesures collectées à l'aide de la méthode de mesure selon l'invention avec les données de la première contraction maximale et/ou du premier relâchement maximal, pour une même série de mesures ou la comparaison avec les données des premières contractions maximales et/ou de relâchements maximaux de séries de mesures précédentes d'un historique de mesures ou des valeurs moyennes de ces mesures précédentes, enregistrées ou stockées dans le dispositif 1 selon l'invention ou dans le dispositif mobile externe. Cette comparaison peut être réalisée à postériori, c'est-à-dire après la fin d'une série de mesures de contractions et/ou de relâchements, mais de préférence, elle est effectuée en temps réel au cours d'une série de mesures. De préférence, pour cette comparaison des données, la méthode de suivi met en oeuvre l'enregistrement des données de contractions et/ou de relâchement, soit dans le ou les dispositifs 1, soit dans le dispositif mobile externe.

La méthode de suivi selon l'invention peut comprendre une étape de communication à l'utilisateur ou l'utilisatrice d'une ou plusieurs représentations graphiques des résultats issus de la comparaison des données. Elle peut également prévoir une étape de réalisation, et la communication à l'utilisateur ou utilisatrice, d'un bilan des contractions et/ou du relâchement musculaire au cours du temps, pour une série de mesures des contractions et/ou de relâchement en tenant compte de l'historique de plusieurs mesures antérieures, et/ou la communication d'un niveau de fatigue du ou des muscles en question.

La méthode d'exercice selon l'invention comprend la mise en oeuvre de la méthode de mesure des contractions et/ou du relâchement, ou de la méthode du suivi de la contraction et/ou du relâchement des muscles selon l'invention.

Comme dans la méthode de mesure des contractions et/ou des relâchements musculaires, dans la méthode d'exercice, l'utilisateur ou utilisatrice peut être à l'initiative des contractions et/ou des relâchements, de leur nombre, amplitude et fréquence. De préférence, et dans le cas d'une mise en oeuvre de l'ensemble de mesure selon l'invention, les contractions et/ou les relâchements s'effectuent en réponse à une demande de contractions musculaires et/ou de relâchement musculaire émanant du dispositif mobile extérieur. A ses fins, le dispositif mobile externe communique à l'utilisateur, ou utilisatrice, une ou des demandes de contractions et/ou des relâchements, des objectifs à atteindre au cours du temps, sous la forme d'un ou plusieurs messages écrits, graphiques ou auditifs, par exemple en affichant une représentation graphique d'une jauge avec une limite d'amplitude de contraction et/ou de relâchement à atteindre, qui reprend une ou plusieurs données de la première contraction maximale et/ou du premier relâchement maximal, ou d'une ou des contractions ultérieures et/ou relâchements ultérieurs, ou bien le niveau, en temps réel, de la force de la contraction et/ou du relâchement en cours.

De préférence, la ou les demandes de contractions et/ou de relâchement de la méthode d'exercice, son ou leur amplitude, durée, nombre et/ou fréquence, tient compte des données de la première contraction maximale et/ou du premier relâchement maximal de la méthode de mesure, et de la fatigue musculaire de l'utilisateur ou utilisatrice identifiée par la méthode de suivi selon l'invention. De préférence, l'amplitude et/ou le nombre et/ou la fréquence des contractions et/ou des relâchements demandées est ou sont adapté(s), avantageusement ajusté(s) en temps réel, en fonction de la contraction maximale et/ou du relâchement maximal enregistré(e) pour la première contraction musculaire et/ou du premier relâchement, et/ou de l'évolution contractions musculaires ultérieures et/ou relâchements ultérieurs, par rapport à cette contraction maximale et/ou un relâchement maximal.

La méthode de mesure et/ou la méthode de suivi et/ou la méthode d'exercice selon l'invention peut, ou peuvent, comprendre une étape de communication du dispositif 1 de mesure selon l'invention, ou du dispositif mobile externe, avec un tiers distant, une entité distante de l'utilisateur ou utilisatrice du dispositif 1 selon l'invention, par exemple un serveur informatique, au travers d'un réseau de communication par exemple téléphonique ou au travers d'Internet. Cette étape peut comprendre la communication à l'utilisateur ou utilisatrice d'informations utiles à la mise en oeuvre des méthodes selon l'invention, ou relatives à la cavité corporelle et/ou les muscles qui la composent. Par exemple, la ou les méthodes peuvent comprendre une étape de communication d'une ou plusieurs indications sur la préparation et/ou l'exécution d'une ou plusieurs contractions, de communication d'indicateurs, par exemple représentatifs de la performance musculaire, l'endurance, les reflexes et/ou la maitrise des contractions, sur la réussite ou l'échec d'un exercice, sur la durée d'exécution du suivi de la contraction musculaire ou de l'exercice effectué.

## Revendications

1. Dispositif (1) de mesure des contractions et/ou du relâchement d'un ou plusieurs muscles d'une cavité corporelle, ledit dispositif (1) comprenant un corps (2) creux venant se positionner dans ladite cavité corporelle, et recouvert d'un revêtement (3) fait, ou comprenant, un matériau bio compatible, ledit corps (2) étant constitué de deux demi coques (7 et 8), chacune étant liées physiquement, de façon permanente et continue lors de l'utilisation dudit dispositif (1), à l'aide de moyens (14) de liaison non compressibles ou déformables, à au moins un capteur (11) de pression, ou partie dudit capteur (11) de pression, disposé dans ledit corps (2).

2. Dispositif (1) selon la revendication 1, dans lequel les moyens (14) de liaison des demi coques (7 et 8) au capteur (11) comprennent, ou sont constituées par, au moins une vis, de préférence deux vis, engageant à la fois une des demi coques (7, 8) et le capteur (11) ou une partie dudit capteur (11).

3. Dispositif (1) selon l'une quelconque des revendications 1 ou 2, dans lequel les demi coques (7, 8) sont montées flottantes l'une par rapport à l'autre, les bords de la première demi coque (7) n'entrant pas en contact avec les bords de la seconde demi coque (8), créant ainsi un espace (9) entre lesdites deux demi coques (7, 8).

4. Dispositif (1) selon l'une quelconque des revendications précédentes, dans lequel ledit dispositif (1) comprend deux capteurs (11) de pression, chaque capteur (11) étant relié, par des moyens (14) de liaison distincts, à une demi coque (7, 8).

5. Dispositif (1) selon l'une quelconque des revendications précédentes, dans lequel le ou les capteur (11) de pression est, ou sont, du type peson et comprend, ou comprennent, un corps (12) fait d'aluminium, et deux jauges de contraintes disposées sur deux surfaces opposées dudit corps (12).

6. Dispositif (1) selon l'une quelconque des revendications précédentes, comprenant en outre des moyens de communication sans fil avec un dispositif mobile externe.

7. Dispositif (1) selon l'une quelconque des revendications précédentes, dans lequel le corps (2) comprend une première partie (4) se trouvant, lors de l'utilisation dudit dispositif (1), dans la cavité corporelle forme, qui est de forme ovoïde, une seconde partie (5) se trouvant, lors de l'utilisation dudit dispositif (1), hors de la cavité corporelle, de forme sphéroïde, ladite première et ladite seconde partie (4, 5) étant reliées entre elles par une partie intermédiaire (6) de forme sensiblement cylindrique.

8. Ensemble de mesure, de suivi des contractions et/ou du relâchement et d'exercice des muscles formant, ou étant compris dans, une cavité corporelle, ledit ensemble comprenant un ou plusieurs dispositifs (1) de mesure selon l'une quelconque des revendications précédentes et un ou plusieurs dispositifs mobiles externes avec lequel ou lesquels ledit ou lesdits dispositifs (1) de mesure communique(nt).

9. Ensemble selon la revendication précédente, dans lequel le ou les dispositifs mobiles externes comprend ou comprennent des moyens de communication avec l'utilisateur ou l'utilisatrice du ou des dispositifs (1) de mesure, et éventuellement également des moyens de communication utilisant un réseau de communication téléphonique ou Internet pour une communication avec un tiers distant.

10. Méthode de mesure de la contraction et/ou du relâchement des muscles d'une cavité corporelle d'un utilisateur, comprenant les étapes suivantes :
- prendre un ou plusieurs dispositifs (1) selon l'une quelconque des revendications 1 à 7, ou l'ensemble de mesure selon les revendications 8 ou 9,
- mettre en marche ledit ou lesdits dispositifs (1) de mesure,
- placer ledit ou lesdits dispositifs (1) de mesure dans une cavité corporelle de l'utilisateur comprenant ou étant formée de muscles,
- identifier et mesurer l'amplitude et/ou de la durée d'une première contraction maximale et/ou d'un premier relâchement maximal desdits muscles,
- identifier, et mesurer au cours du temps, l'amplitude et/ou la durée et/ou la fréquence d'une ou plusieurs contractions ultérieures et/ou d'un ou plusieurs relâchement ultérieurs,
l'amplitude et/ou de la durée de ladite première contraction maximale et/ou dudit premier relâchement maximal et l'amplitude et/ou la durée et/ou la fréquence de ladite ou desdites contractions ultérieures et/ou relâchements ultérieurs, constituant des données de mesures,
- communiquer et présenter, en temps réel, à l'utilisateur lesdites données de mesures.

11. Méthode de mesure selon la revendication précédente, comprenant en outre une étape de communication par le ou les dispositifs (1) de mesure de son ou leur état de fonctionnement et/ou une étape de calibration du ou desdits dispositifs (1) de mesure avant et/ou après sa ou leur mise en place dans la cavité corporelle.

12. Méthode de suivi de la contraction et/ou du relâchement des muscles d'une cavité corporelle comprenant la mise en oeuvre de la méthode de mesure selon l'une quelconque des revendications 10 ou 11 et une étape de comparaison des données de mesures, collectées au cours du temps à l'aide de ladite méthode de mesure, avec les données de la première contraction maximale et/ou du relâchement maximal, pour une même série de mesures, ou la comparaison avec les données des premières contractions maximales et/ou de relâchements maximaux de séries de mesures précédentes d'un historique de mesures ou d'une ou plusieurs valeurs moyennes de premières contractions maximales et/ou de relâchements maximaux desdites mesures précédentes.

13. La méthode de suivi selon la revendication précédente, comprenant en outre une étape de communication à l'utilisateur d'une ou plusieurs représentations écrites, graphiques et/ou auditives, des résultats issues de l'étape de comparaison des données.

14. Méthode d'exercice des muscles d'une cavité corporelle comprenant la mise en oeuvre de la méthode de mesure selon l'une quelconque des revendications 10 ou 11 ou de la méthode de suivi selon l'une quelconque des revendications 12, ou 13, et comprenant une étape d'adaptation de l'amplitude et/ou la durée et/ou le nombre et/ou fréquence des contractions et/ou relâchements effectuées par, ou demandées à, l'utilisateur en fonction des données de la première contraction maximale et/ou premier relâchement maximal et/ou de la fatigue musculaire de l'utilisateur identifiée par ladite méthode de suivi.

15. La méthode de mesure selon l'une quelconque des revendications 10 ou 11 et/ou la méthode de suivi selon l'une quelconque des revendications 12 ou 13 et/ou la méthode d'exercice selon la revendication 14, comprenant en outre une étape de communication du ou des dispositifs (1) de mesure, ou du dispositif mobile externe, avec un tiers distant de l'utilisateur pour la préparation et/ou l'exécution d'une ou plusieurs étapes desdites méthodes de mesure, de suivi et/ou d'exercice, ou pour la communication d'indicateurs représentatifs de la performance musculaire de l'utilisateur.

## Patentansprüche

1. Vorrichtung (1) zur Messung der Kontraktionen und/oder der Entspannung von einem oder mehreren Muskeln einer Körperhöhle, wobei die Vorrichtung (1) einen Hohlkörper (2) umfasst, der in der Körperhöhle positioniert wird und mit einer Beschichtung (3) aus einem biokompatiblen Material versehen ist oder dieses umfasst, wobei der Körper (2) aus zwei Halbschalen (7 und 8) besteht, die jeweils physisch dauerhaft und kontinuierlich während der Verwendung der Vorrichtung (1) mit Hilfe von nicht komprimierbaren oder verformbaren Verbindungsmitteln (14) mit mindestens einem Drucksensor (11) oder einem Teil des Drucksensors (11) verbunden sind, der in dem Körper (2) angeordnet ist.

2. Vorrichtung (1) nach Anspruch 1, wobei die Verbindungsmittel (14) der Halbschalen (7 und 8) mit dem Sensor (11) mindestens eine Schraube, vorzugsweise zwei Schrauben, umfassen oder aus diesen bestehen, die sowohl in eine der Halbschalen (7, 8) als auch in den Sensor (11) oder in einen Teil des Sensors (11) eingreifen.

3. Vorrichtung (1) nach einem der Ansprüche 1 oder 2, wobei die Halbschalen (7, 8) relativ zueinander schwimmend montiert sind, wobei die Ränder der ersten Halbschale (7) die Ränder der zweiten Halbschale (8) nicht berühren, wodurch ein Raum (9) zwischen den beiden Halbschalen (7, 8) entsteht.

4. Vorrichtung (1) nach einem der vorhergehenden Ansprüche, wobei die Vorrichtung (1) zwei Drucksensoren (11) umfasst, wobei jeder Sensor (11) über verschiedene Verbindungsmittel (14) mit einer Halbschale (7, 8) verbunden ist.

5. Vorrichtung (1) nach einem der vorhergehenden Ansprüche, wobei der oder die Drucksensoren (11) vom Typ Federwaage ist oder sind und einen Körper (12) aus Aluminium sowie zwei Dehnungsmessstreifen umfasst oder umfassen, die auf zwei gegenüberliegenden Oberflächen des Körpers (12) angeordnet sind.

6. Vorrichtung (1) nach einem der vorhergehenden Ansprüche, die ferner Mittel zur drahtlosen Kommunikation mit einer externen mobilen Vorrichtung umfasst.

7. Vorrichtung (1) nach einem der vorhergehenden Ansprüche, wobei der Körper (2) einen ersten Teil (4) umfasst, der sich bei der Verwendung der Vorrichtung (1) in der Körperhöhle befindet, der eiförmig ist, einen zweiten Teil (5), der sich bei der Verwendung der Vorrichtung (1) außerhalb der Körperhöhle befindet, der kugelförmig ist, wobei der erste und der zweite Teil (4, 5) durch einen Zwischenteil (6) verbunden sind, der im Wesentlichen zylindrisch ist.

8. Anordnung zur Messung, zur Überwachung der Kontraktion und/oder der Entspannung und des Trainings von Muskeln, die eine Körperhöhle bilden oder darin enthalten sind, wobei die Anordnung eine oder mehrere Messvorrichtungen (1) nach einem der vorhergehenden Ansprüche und eine oder mehrere externe mobile Vorrichtungen umfasst, mit dem oder denen die Messvorrichtung(en) (1) kommuniziert/kommunizieren.

9. Anordnung nach vorhergehendem Anspruch, wobei die externe(n) mobile(n) Vorrichtung(en) Mittel zur Kommunikation mit dem Benutzer oder der Benutzerin der Messvorrichtung(en) (1) und gegebenenfalls auch Mittel zur Kommunikation unter Verwendung eines Telefonkommunikationsnetzes oder des Internets für eine Kommunikation mit einem entfernten Dritten umfasst/umfassen.

10. Verfahren zum Messen der Kontraktion und/oder der Entspannung von Muskeln einer Körperhöhle eines Benutzers, das die folgenden Schritte umfasst:
- Bereitstellen einer oder mehrerer Vorrichtungen (1) nach einem der Ansprüche 1 bis 7 oder der Messanordnung nach den Ansprüchen 8 oder 9,
- Einschalten der Messvorrichtung(en) (1),
- Platzieren der Messvorrichtung(en) (1) in einer Körperhöhle des Benutzers, die Muskeln umfasst oder aus ihnen besteht,
- Identifizieren und Messen der Amplitude und/oder der Dauer einer ersten maximalen Kontraktion und/oder einer ersten maximalen Entspannung der Muskeln,
- Identifizieren und Messen der Amplitude und/oder der Dauer und/oder der Frequenz einer oder mehrerer nachfolgender Kontraktionen und/oder einer oder mehrerer nachfolgender Entspannungen im Laufe der Zeit,
wobei die Amplitude und/oder die Dauer der ersten maximalen Kontraktion und/oder der ersten maximalen Entspannung und die Amplitude und/oder die Dauer und/oder die Frequenz der nachfolgenden Kontraktion(en) und/oder nachfolgenden Entspannung(en) die Messdaten darstellen,
- Kommunizieren und Darstellen der genannten Messdaten gegenüber dem Benutzer in Echtzeit.

11. Messverfahren nach vorhergehendem Anspruch, das ferner einen Schritt umfasst, bei dem die Messvorrichtung(en) (1) ihren Betriebszustand bzw. ihre Betriebszustände mitteilen, und/oder einen Schritt, bei dem die Messvorrichtung(en) (1) vor und/oder nach ihrer Platzierung in der Körperhöhle kalibriert wird bzw. werden.

12. Verfahren zur Überwachung der Kontraktion und/oder der Entspannung der Muskeln einer Körperhöhle, umfassend die Durchführung des Messverfahrens nach einem der Ansprüche 10 oder 11 und einen Schritt des Vergleichens der Messdaten, die im Laufe der Zeit mithilfe des Messverfahrens gesammelt wurden, mit den Daten der ersten maximalen Kontraktion und/oder der maximalen Entspannung für eine gleiche Messreihe oder des Vergleichens mit den Daten der ersten maximalen Kontraktionen und/oder der maximalen Entspannungen früherer Messreihen aus einer Messhistorie oder mit einem oder mehreren Durchschnittswerten der ersten maximalen Kontraktionen und/oder der maximalen Entspannungen der früheren Messungen.

13. Überwachungsverfahren nach vorhergehendem Anspruch, das ferner einen Schritt umfasst, bei dem Benutzer eine oder mehrere schriftliche, grafische und/oder auditive Darstellungen der Ergebnisse aus dem Schritt des Datenvergleichs mitgeteilt werden.

14. Verfahren zum Trainieren der Muskeln einer Körperhöhle, umfassend die Durchführung des Messverfahrens nach einem der Ansprüche 10 oder 11 oder des Überwachungsverfahrens nach einem der Ansprüche 12 oder 13, und umfassend einen Schritt des Anpassens der Amplitude und/oder der Dauer und/oder der Anzahl und/oder der Frequenz der Kontraktionen und/oder der Entspannungen, die vom Benutzer ausgeführt oder von ihm verlangt werden, in Abhängigkeit von den Daten der ersten maximalen Kontraktion und/oder der ersten maximalen Entspannung und/oder der Muskelermüdung des Benutzers, die durch das Überwachungsverfahren identifiziert wird.

15. Messverfahren nach einem der Ansprüche 10 oder 11 und/oder Überwachungsverfahren nach einem der Ansprüche 12 oder 13 und/oder das Trainingsverfahren nach Anspruch 14, umfassend ferner einen Schritt der Kommunikation der Messvorrichtung(en) (1) oder der externen mobilen Vorrichtung mit einem vom Benutzer entfernten Dritten zur Vorbereitung und/oder Durchführung eines oder mehrerer Schritte der Mess-, Überwachungs- und/oder Trainingsverfahren oder zur Kommunikation von Indikatoren, die für die Muskelleistung des Benutzers repräsentativ sind.

## Claims

1. Device (1) for measuring contractions and/or the relaxation of one or several muscles of a body cavity, said device (1) comprising a hollow body (2) which is for positioning in said body cavity, and which is covered by a coating (3) made of, or comprising, a biocompatible material, said body (2) being formed by two half-shells (7 and 8) which are each physically connected, permanently and continuously during the use of said device (1), using non-compressible or deformable connecting means (14), to at least one pressure sensor (11), or part of said pressure sensor (11), arranged in said body (2).

2. Device (1) according to claim 1, wherein the connecting means (14) of the two half-shells (7 and 8) to the sensor (11) comprise, or are made up of, at least one screw, preferably two screws, engaging both one of the half-shells (7, 8) and the sensor (11) or a part of said sensor (11).

3. Device (1) according to any one of claims 1 or 2, wherein the half-shells (7, 8) are mounted floating relative to one another, the edges of the first half-shell (7) not coming into contact with the edges of the second half-shell (8), thus creating a space (9) between the two half-shells (7, 8).

4. Device (1) according to any one of the preceding claims, wherein said device (1) comprises two pressure sensors (11), each sensor (11) being coupled, by separate connecting means (14), to a half-shell (7, 8).

5. Device (1) according to any one of the preceding claims, wherein the pressure sensor(s) (11) is, or are, of the weight indicator type and comprises(s) a body (12) made from aluminum and two strain gauges positioned on two opposite surfaces of the body (12).

6. Device (1) according to any one of the preceding claims, further comprising wireless communication means with an external mobile device.

7. Device (1) according to any one of the preceding claims, wherein the body (2) comprises a first part (4) located, during the use of said device (1), in the bodily cavity form, which is ovoid, a second part (5) located, during the use of said device (1), outside the bodily cavity, with a spheroid shape, said first and said second parts (4, 5) being coupled to one another by an intermediate part (6) with a substantially cylindrical shape.

8. Measuring assembly for monitoring contractions and/or the relaxation and exercise of muscles forming, or being comprised in, a bodily cavity, the assembly comprising one or several measuring devices (1) according to any one of the preceding claims and one or several external mobile devices with which said measuring device(s) (1) communicate(s).

9. Assembly according to the preceding claim, wherein the external mobile device(s) comprise(s) means of communication with the user of the measuring device(s) (1), and optionally also means of communication using a telephone or Internet communication network for communication with a remote third party.

10. Method for measuring the contraction and/or the relaxation of the muscles of a bodily cavity of a user, comprising the following steps:
- taking one or several measuring devices (1) according to any one of claims 1 to 7, or the measuring assembly according to claims 8 or 9,
- starting said measuring device(s) (1),
- placing said measuring device(s) (1) in a bodily cavity of the user comprising or being formed by muscles,
- identifying and measuring the amplitude and/or the duration of a first maximal contraction and/or a first maximal relaxation of said muscles,
- identifying and measuring, over time, the amplitude and/or the duration and/or the frequency of one or several subsequent contractions and/or subsequent relaxations,
the amplitude and/or the duration of said first maximal contraction and/or said first maximal relaxation and the amplitude and/or the duration and/or the frequency of said subsequent contraction(s) and/or subsequent relaxations constituting measurement data, and
- communicating and displaying, in real time, the measurement data to the user.

11. Method according to the preceding claim, further comprising a step for communication by the measuring device(s) (1) of its or their operating state and/or a step for calibration of said measuring device(s) (1), before and/or after it or they are placed in the bodily cavity.

12. Method for monitoring the contraction and/or the relaxation of the muscles of a bodily cavity comprising the implementation of the measuring method according to any one of claims 10 or 11, and a step for comparing measuring data, collected over time using said measuring method, with the data of the first maximal contraction and/or the first maximal relaxation, for a same series of measurements, or the comparison with the data of the first maximal contractions and/or maximal relaxations of preceding series of measurements from a measurement history or one or several average values of first maximal contractions and/or maximal relaxations of said preceding measurements.

13. Method according to the preceding claim, further comprising a step for communicating, to the user, one or several written, graphic and/or auditory representations of the results from the step for comparison of the data.

14. Method for exercising muscles of a bodily cavity comprising implementing the measuring method according to any one of claims 10 or 11 or the monitoring method according to any one of claims 12 or 13, and comprising a step for adapting the amplitude and/or the duration and/or the number and/or the frequency of the contractions and/or relaxations done by, or requested from, the user as a function of the data from the first maximal contraction and/or first maximal relaxation and/or the muscle fatigue of the user identified by said monitoring method.

15. The measuring method according to any one of claims 10 or 11 and/or the monitoring method according to any one of claims 12 or 13 and/or the exercise method according to claim 14, further comprising a step for communication by the measuring device(s) (1), or the external mobile device, with a third party remote from the user in order to prepare and/or execute one or several steps of the measuring, monitoring and/or exercise methods, or for the communication of indicators representative of the muscle performance of the user.
